# EUROPÄISCHE PATENTSCHRIFT

(11) **EP 4 343 324 B1**
(45) Veröffentlichungstag und Bekanntmachung des Hinweises auf die Patenterteilung: **19.11.2025**
(21) Anmeldenummer: 22197689.7
(22) Anmeldetag: 26.09.2022
(51) Int. Cl.: G01N 33/18, C02F 1/42, B01J 39/00, B01J 41/00, B01J 47/028

(54) **VERFAHREN ZUM BESTIMMEN DES TOC-GEHALTES EINER WÄSSRIGEN LÖSUNG UND ENTSPRECHENDE VORRICHTUNG**
METHOD FOR DETERMINING THE TOC CONTENT OF AN AQUEOUS SOLUTION AND CORRESPONDING DEVICE
PROCÉDÉ DE DÉTERMINATION DE LA TENEUR EN TOC D'UNE SOLUTION AQUEUSE ET DISPOSITIF CORRESPONDANT

(43) Veröffentlichungstag der Anmeldung: 27.03.2024
(73) Patentinhaber: MionTec GmbH, 51377 Leverkusen (DE)
(72) Erfinder: Mauer, Dieter, 51377 Leverkusen (DE)
(74) Vertreter: Erbacher, Martin

(56) Entgegenhaltungen:
- EP-A2- 0 135 685
- WO-A1-2009/147511
- DE-A1- 102019 124 843
- US-B1- 6 444 474

## Beschreibung

Die Erfindung geht aus von einem Verfahren zum Bestimmen des TOC-Gehaltes einer wässrigen Lösung nach Durchlaufen einer Ionenaustauscher-Vollentsalzungsstraße, aufweisend die Schritte:
a) Messen einer ersten Leitfähigkeit der wässrigen Lösung;
b) Oxidieren von in der wässrigen Lösung enthaltenem organischen Kohlenstoff zu CO2 und/oder H+ und/oder HCO3- und/oder Na+ + HCO3-;
c) Messen einer zweiten Leitfähigkeit der wässrigen Lösung;
d) Bestimmen des TOC-Gehalts der wässrigen Lösung aus den beiden gemessenen Leitfähigkeiten.

Die Messung des Gehaltes an totalem organischen Kohlenstoff (TOC, an dieser Stelle gleichbedeutend mit dem gelösten organischen Kohlenstoff, DOC) besteht immer aus der Umwandlung von organischem C durch eine Oxidation in Kohlensäure als Mischung von CO₂ oder H⁺ + HCO₃⁻ oder Na⁺ + HCO₃⁻. Die Oxidation zur und auch der Nachweis der entstandenen Kohlensäure kann dabei auf unterschiedliche Weise geschehen. Die bislang genaueste Technik - auch die deutlich teuerste - benutzt einen spektroskopischen Nachweis des CO₂ im infraroten Wellenlängenbereich. Hierzu wird durch Zugabe von analytisch reiner Säure die durch die Oxidation entstandene Kohlensäure komplett in die CO₂-Form umgesetzt, da HCO₃⁻ über den spektroskopischen Nachweis nicht zugänglich ist.

Den umgekehrten Weg geht demgegenüber der Nachweis über die Leitfähigkeit. Hier ist nur HCO₃⁻ in Verbindung mit H⁺ oder Na⁺ nachweisbar. Allerdings erlaubt die Anwendung des Massenwirkungsgesetzes (MWG) und der Henderson-Hasselbalch-Gleichung (HH-Gl.) die Umrechnung der gemessenen Leitfähigkeit in sowohl den HCO₃⁻/H⁺-Anteil, als auch die Bestimmung des über diese Gleichungen den nicht per Leitfähigkeit messbaren CO₂-Anteil. Die wichtige Voraussetzung, dass dies ausreichend genau funktioniert, ist eine sehr geringe Gesamt-Leitfähigkeit (mit Bezug auf die erfindungsgemäße Anwendung im Wesentlichen durch zusätzliches Na⁺) von höchstens ca. 2 µS/cm. Dieser Wert entspricht der typischen Herstellerangabe für kommerzielle TOC-Messgeräte nach Leitfähigkeitsmethode. In der Praxis treten deutliche Fehlanzeigen bereits bei viel geringeren Leitfähigkeiten als dieser auf, deren Ausmaß vom pH-Wert abhängt. Dieser liegt nach einer Ionenaustauscher-Vollentsalzungsstraße (VE-Straße) meist oberhalb 8, teilweise bis zu 9 und manchmal sogar höher.

Aus dem Stand der Technik sind die folgend beschriebenen Methoden zur Bestimmung des TOC-Gehalts bekannt. Die wesentlichen Schritte bestehen dabei in der Oxidation, dem Nachweis der entstandenen Kohlensäure sowie der c)Unterdrückung von Nullsignalen oder Fehlern.

Zum einen ist die katalytische Oxidation im Ofen bekannt. Alle TOC-Messungen nutzen eine Oxidation des C zum CO2. Bei teuren Geräten wird dies in einem bis zu 1200 °C heißen Ofen unter Nutzung von Katalysatoren durchgeführt. Bei diesen Bedingungen ist die Vollständigkeit der Umsetzung sehr gut, diese Technik ist jedoch auch am teuersten. Ferner ist die nasschemische Oxidation bekannt. Die nasschemische Oxidation wird oft mit Persulfat durchgeführt. Hier spielt zum einen die Reinheit der Chemikalien eine große Rolle und zum anderen benötigt die Methode einen hohen apparativen Aufwand. Die praktische Relevanz für automatisierte Messsysteme ist ohnehin vernachlässigbar. Des Weiteren existiert als weitere Methode die Oxidation per UV-Bestrahlung. Dies ist die günstigste Methode, welche durch UV-Bestrahlung des Probenwassers funktioniert. Hier bieten sich Niederdruck-UV-Lampen an, welche bei 256 und bei 185 nm starke Emissionslinien besitzen. Diese Strahlung bildet im Wasser OH⁻-Radikale, welche dann ihrerseits die eigentliche Oxidation des C zum CO₂ durchführen. Diese Technik oxidiert unter Umständen nicht ganz vollständig, ist also eher ungenauer im Messwert, dafür aber sehr preiswert.

Für den Nachweis der entstandenen Kohlensäure existiert zum einen die spektroskopische Methode. Prinzipiell lässt sich nach jedem Oxidationsverfahren CO₂ aus der Probe ausstrippen und über ein Spektrometer mit NDIR-Detektor nachweisen. Dies kann Bestimmungsgrenzen um wenige ppb TOC erreichen. Dies ist zwar die genaueste, aber auch die aufwendigste Methode. Des Weiteren existiert für den Nachweis der entstandenen Kohlensäure die Methode der Differenzleitfähigkeit. Insbesondere bei der Oxidation mit einer UV-Lampe wird alternativ die zusätzlich durch HCO₃⁻ und die zugehörigen H⁺-Ionen entstehende Leitfähigkeit gegenüber der Leitfähigkeit vor der UV-Lampe gemessen. Mit dem elektrisch nicht leitfähigen CO₂ steht das HCO₃⁻ in einem Gleichgewicht, welches sich prinzipiell berechnen lässt. Die Genauigkeit dieser Berechnung ist sehr gut, wenn außer der Kohlensäure keine weiteren Bestandteile im Wasser vorliegen. Diese Methode wird daher meist im Reinstwasserbereich eingesetzt, wo die Leitfähigkeit vor der Oxidation typischerweise bei << 1 µS/cm liegt. Durch Differenzbildung mit dieser Leitfähigkeit vor der UV-Zelle kann ein gewisser Einfluss sonstiger Ionen kompensiert werden, aber die Ungenauigkeit steigt mit steigender Leitfähigkeit vor der UV-Zelle deutlich an. Die Ablaufqualität von Ionenaustauscher-Vollentsalzungsstraßen zeigt am Anfang und am Ende der Beladung oft Werte bis ≈ 5 µS/cm, was diese Messung deutlich fehleranfälliger werden lässt, da der stärkste Einfluss auf die zusätzliche Leitfähigkeit durch das H⁺ erfolgt, welches im leicht alkalischen Ablauf der VE-Straßen weitgehend wegneutralisiert wird.

Für die Unterdrückung von Nullsignalen oder Fehlern ist bei den spektroskopischen Verfahren die Trennung von TIC und TOC in der Probe bekannt. Nach der Oxidation kann HCO₃⁻/CO₂, welches vorher bereits anwesend war (TIC, total inorganic carbon) nicht mehr von dem aus dem TOC neu erzeugten HCO₃⁻/CO₂ unterschieden werden. Geräte mit spektroskopischem Nachweis besitzen daher vor der Oxidation eine Ansäuerungsstufe mit Strippung der dabei ausgasenden CO₂. Es gilt die Anforderung der CO₂-Freiheit an die Strippluft und eine hohe Reinheitsanforderung an die Säure. Wenn im Reinstwasserbereich gemessen werden soll, kann der vorher vorhandene TIC meist vernachlässigt werden. Insbesondere im Ablauf einer VE-Straße liegt sehr wenig Kohlensäure (TIC) vor, da typischerweise Na⁺ und OH⁻ dominieren. In dieser Anwendung ist eine Unterdrückung von neben dem TOC vorhandenem TIC also meist nicht notwendig.

Im Gegensatz zu den Geräten mit spektroskopischem Nachweis erzeugen Fremdionen außerhalb des TIC bei der Differenzleitfähigkeitsmethode ein Störsignal. Die Na⁺-Ionen und deren zugehörige OH⁻ erzeugen zum Beispiel eine spürbare Leitfähigkeit bereits vor der UV-Zelle, welche sich typisch im Bereich 0,1 bis 5 µS/cm bewegt. Bei Leitfähigkeitsanstiegen durch die UV-Lampe von 1 bis 2 µS/cm ist klar, dass dieser Einfluss nicht vernachlässigt werden kann. Hier stellt sich das durch die erfindungsmäßige Methode umgangene Problem deutlich dar. Es wird sogar noch einmal zusätzlich schwieriger, da die Gleichungen, welche aus der einzig messbaren HCO₃⁻-Konzentration das parallel vorhandene, aber nicht messbare CO₂ ermitteln, bei Anwesenheit von OH⁻ deutlich falsch werden. Hier sind Ungenauigkeiten um Faktoren möglich. Aus der Druckschrift WO 2009/147511 A1 ist ein Verfahren zur Analyse der Reinheit von Wasser am Ausgang einer Reinigungsvorrichtung bekannt, bei welchem eine Flüssigkeit am Ausgang eines Filtermittels durch eine Widerstandsmesszelle durchgeleitet wird, um den spezifischen Widerstand zu bestimmen. Ferner wird ein Teil der Flüssigkeit Oxidationsmitteln einer bestimmten Anzahl von deutlich unterschiedlichen Zeitspannen ausgesetzt und jeweils der spezifische Widerstand der Flüssigkeit bestimmt. Das Verfahren umfasst ferner das Berechnen der Menge der in der gereinigten Flüssigkeit enthaltenen organischen Verbindungen anhand der unterschiedlichen gemessenen spezifischen Widerstände. Ähnliche Verfahren sind außerdem bekannt aus den Druckschriften EP O 135 685 A2, US 6 444 474 B1 und DE 10 2019 124 843 A1.

Es besteht daher ein Bedürfnis danach, die störenden Leitfähigkeitsbeiträge zu reduzieren, ohne die organischen Gehalte maßgeblich zu beeinflussen. Damit wäre die preiswertere Leitfähigkeits-Messtechnik einsetzbar, ohne die deutlich teurere spektroskopische Technik verwenden zu müssen.

Es ist daher die objektiv technische Aufgabe der Erfindung, ein Verfahren bzw. eine Vorrichtung zum Bestimmen des TOC-Gehaltes einer wässrigen Lösung derart zu verbessern, dass damit die Bestimmung des TOC-Gehalts zum einen wenig fehleranfällig und zum anderen preiswert ist.

Die Aufgabe wird gelöst durch ein Verfahren und eine Vorrichtung mit den Merkmalen der unabhängigen Ansprüche. Vorteilhafte Ausgestaltungen sind in den Unteransprüchen angegeben.

Demgemäß ist vorgesehen, dass vor dem Messen der ersten Leitfähigkeit ein Austauschen von in der wässrigen Lösung enthaltenen anorganischen Kationen, wie beispielsweise Na+, K+ usw., gegen H+-Ionen erfolgt, wobei das Austauschen ein Umwandeln des NaOH⁻- und/oder des NaHCO₃-Anteils der wässrigen Lösung in H₂O und CO₂ mittels eines Kationenaustauschers in H⁺-Form umfasst.

Die vorliegende Erfindung betrifft damit eine neuartige Technologie für die zuverlässige und trotzdem preisgünstige Messung des Gehaltes an organischem Kohlenstoff (TOC) nach Ionenaustauscher-Vollentsalzungsstraßen. Die Erfindung erreicht dieses Ziel durch die Entfernung der durch eine VE-Straße typischerweise übrig gelassenen anorganischen Kationen vor dem Messen der Differenzleitfähigkeit. Die Erfindung überwindet somit das Problem der prinzipiell kostengünstigen Methode nach der Differenzleitfähigkeit über einer oxidierenden UV-Lampe, dass die Leitfähigkeit vor der UV-Zelle durch den Schlupf der Kationenaustauschers der VE-Straße so hoch sein kann, dass die Mess- und Rechenmethoden zur Ermittlung des Kohlensäuregehaltes nach der UV-Lampe fehlerhafte TOC-Werte liefern. Das erfindungsgemäße Verfahren benutzt zur Lösung des Problems einen der UV-Oxidationszelle vorgeschalteten Kationenaustauscher, um den störenden NaOH⁻-Anteil in diesem Zulaufwasser in Wasser umzuwandeln, so dass die formelmäßige Auswertung der Leitfähigkeitsdifferenz deutlich weniger fehleranfällig wird und trotzdem die preiswerte Messtechnik genutzt werden kann. Der Kern der Erfindung zur Messung von TOC-Gehalten nach VE-Straßen oder leicht alkalischen und mineralsäurefreien Probenströmen besteht damit in der Bereitstellung eines Verfahrens bzw. einer Anordnung umfassend einen Kationenaustauscher, eine Leitfähigkeitsmesszelle, eine UV-Lampe in einer Durchlaufzelle und einer weiteren Leitfähigkeitsmessung zusammen mit einer Auswertemethode, welche NaOH⁻-und/oder NaHCO₃-Anteile nur noch in winzigen und vor allem leicht berechenbaren Korrekturen zu berücksichtigen braucht.

Die Erfindung erreicht somit auf einfachstem Wege eine für die Beurteilung einer VE-Straße ausreichende Genauigkeit bzw. Bestimmungsgrenze. Ferner ist sie auf alle Anwendungen übertragbar, in denen der TOC-Gehalt in einer geringfügig alkalischen Umgebung gemessen werden soll. Damit stellt die erfindungsgemäße Technologie eine sehr günstige Verbesserung und Weiterentwicklung für sehr viele Messverfahren in Ionenaustauscher-VE-Anlagen und leicht alkalischen Lösungen dar.

Es kann vorgesehen sein, dass mittels des Kationenaustauschers das Na⁺ in der wässrigen Lösung gegen H⁺ ausgetauscht wird, welches überschüssige OH⁻-Ionen zu Wasser neutralisiert.

Beispielsweise kann die erste Leitfähigkeit der wässrigen Lösung zwischen ≤ 0,1 und 0,2 µS/cm betragen. Demgegenüber kann die zweite Leitfähigkeit der wässrigen Lösung (nach der UV-Zelle) zwischen 0,5 und 2 µS/cm betragen.

Es kann vorgesehen sein, dass die wässrige Lösung nach dem Oxidieren Konzentrationen von Na⁺ und/oder OH⁻ von jeweils geringer als 5 µmol/l aufweist.

Ferner kann vorgesehen sein, dass der Kationenaustauscher ein starksaurer oder ein schwachsaurer Kationenaustauscher ist.

Beispielsweise kann das Oxidieren des in der wässrigen Lösung enthaltenen organischen Kohlenstoffs mittels UV-Bestrahlung erfolgen, wobei die UV-Bestrahlung mittels zumindest einer Niederdruck-UV-Lampe erfolgen kann.

Es kann vorgesehen sein, dass das Messen der ersten und der zweiten Leitfähigkeit der wässrigen Lösung jeweils die Eigenleitfähigkeit von H₂O herausgerechnet wird. Da dadurch die Eigenleitfähigkeit von Wasser von 0,055 µS/cm (errechnet mit 10⁻⁷ mol/l H⁺ und 10⁻⁷ mol/l OH⁻ in pH-neutralem Wasser) abgezogen wird, bleiben noch typische Leitfähigkeitsanteile von 0,05 bis 0,15 µS/cm für das störende NaOH. Diese Werte sind dann so klein, dass sie nur noch Fehlanzeigen von 5 bis 10 ppb TOC erzeugen (bei Messwerten von z.B. 100 bis 200 ppb TOC).

Es kann vorgesehen sein, dass das Verfahren ferner umfasst: Messen des pH-Werts der wässrigen Lösung nach dem Oxidieren des in der wässrigen Lösung enthaltenen organischen Kohlenstoffs; Berechnen eines theoretischen pH-Werts aus der gemessenen zweiten Leitfähigkeit; Bilden eines Mittelwerts aus dem gemessenen pH-Wert und dem berechneten theoretischen pH-Wert; Verwenden des gemittelten pH-Werts für die Berechnung der Leifähigkeitsdifferenz. Durch Einbeziehung des pH-Wertes mit seiner redundanten Möglichkeit die H⁺-Konzentration rechnerisch zu ermitteln, kann die Genauigkeit des Verfahrens noch weiter erhöht werden. Zur Erreichung höherer Genauigkeit können analog zu obiger Darstellung auch an beiden Messpositionen, vor und nach dem Oxidieren, pH-Messungen zugefügt werden.

Insgesamt erreicht man dadurch eine Bestimmungsgrenze um 10 ppb, was für die Beurteilung einer Vollentsalzungsstraße völlig ausreicht. Die TOC-Abgabe solcher Anlagen liegt meist zwischen 20 bis 300 ppb, was dem Ziel-Messbereich des erfindungsgemäßen Verfahrens entspricht. "Schlechte" Anlagen zeigen meist TOC-Werte von über 200 ppb, so dass Alarmierungen in diesem Messbereich gut genug auflösend sind. "Gute" Anlagen laufen demgegenüber mit TOC-Werten von unter 100 ppb ab. Nachdem der Störeinfluss der NaOH eliminiert ist, können die standardmäßigen Formeln zur Umrechnung von Leitfähigkeit in HCO₃⁻ und über die Henderson-Hasselbalch-Gleichung dann auch die Konzentration der zugehörigen CO₂ errechnet werden, ohne deutlich Ungenauigkeit in Kauf nehmen zu müssen.

Die Erfindung betrifft ferner eine Vorrichtung zum Bestimmen des TOC-Gehaltes einer wässrigen Lösung nach Durchlaufen einer Ionenaustauscher-Vollentsalzungsstraße, mit zumindest einer Messeinrichtung zum Messen der Leitfähigkeit der wässrigen Lösung, welche eine erste Messposition zum Messen einer ersten Leitfähigkeit und eine zweite Messposition zum Messen einer zweiten Leitfähigkeit der wässrigen Lösung aufweist, wobei die erste Messposition stromaufwärts der zweiten Messposition angeordnet ist, und mit einer zwischen den Messpositionen angeordneten Oxidationseinrichtung zum Oxidieren von in der wässrigen Lösung enthaltenem organischen Kohlenstoff zu CO₂ und/oder H⁺ und/oder HCO₃⁻ und/oder Na⁺ + HCO₃⁻, sowie mit einer Auswerteinheit zum Berechnen der Differenz zwischen der ersten und der zweiten gemessenen Leitfähigkeit und zum Bestimmen des TOC-Gehalts der wässrigen Lösung aus der berechneten Leitfähigkeitsdifferenz, wobei die Vorrichtung ferner eine stromaufwärts der ersten Messposition angeordneten Kationenaustauscher in H+-Form zum Austauschen von in der wässrigen Lösung enthaltenen anorganischen Kationen, wie beispielsweise Na⁺, K⁺ usw., gegen H⁺-Ionen aufweist.

Es kann vorgesehen sein, dass der Kationenaustauscher ein starksaurer oder ein schwachsaurer Kationenaustauscher ist.

Es ist denkbar, dass die Oxidationseinrichtung zumindest eine Niederdruck-UV-Lampe aufweist.

Die Vorrichtung kann ferner eine stromabwärts der Oxidationseinrichtung angeordnete Einrichtung zum Messen des pH-Werts der wässrigen Lösung aufweisen, wobei die Auswerteinheit ferner zum Berechnen eines theoretischen pH-Werts aus der gemessenen zweiten Leitfähigkeit, zum Bilden eines Mittelwerts aus dem gemessenen pH-Wert und dem berechneten theoretischen pH-Wert sowie zum Verwenden des gemittelten pH-Werts für die Berechnung der Leifähigkeitsdifferenz eingerichtet sein kann.

Darüber hinaus kann vorgesehen sein, dass die Vorrichtung eine stromaufwärts der Austauscheinrichtung angeordnete Ionenaustauscher-Vollentsalzungsstraße aufweist.

Weitere Eigenschaften, Vorteile und Merkmale der Erfindung sind in der folgenden Beschreibung bevorzugter Ausführungen der Erfindung anhand der beiliegenden Zeichnungen erkennbar, in denen zeigen:
- Fig. 1: ein Ablaufdiagramm einer Methode der katalytischen Oxidation mit spektroskopischem Nachweis;
- Fig. 2: ein Ablaufdiagramm einer Methode der Differenzleitfähigkeit über UV-Lampe;
- Fig. 3: ein Ablaufdiagramm einer Ausführungsform des erfindungsgemäßen Verfahrens;
- Fig. 4: eine Tabelle mit Erwartungswerten für die zusätzliche Gesamtkohlensäure nach Oxidation durch eine UV-Zelle;
- Fig. 5: ein Diagramm hinsichtlich des Zusammenhangs zwischen Leitfähigkeit und pH-Wert in drei verschiedenen Bereichen;
- Fig. 6: ein Diagramm hinsichtlich der Erweiterung des auswertbaren Leitfähigkeitsbereich bis < 1 µS/cm;
- Fig. 7: ein Diagramm hinsichtlich eines nicht erkennbaren Zusammenhangs zwischen Leitfähigkeit und pH-Wert ohne Einsatz eines Kationenaustauschers;
- Fig. 8: ein Diagramm hinsichtlich des deutlichen Zusammenhangs zwischen Leitfähigkeit und pH-Wert mit Einsatz eines Kationenaustauschers;
- Fig. 9: ein Diagramm mit einem Vergleich zwischen einer gemessenen Leitfähigkeit nach UV-Oxidation ohne Einsatz eines Kationenaustauschers einerseits mit kommerziellen Messgeräten gemessenen Leitfähigkeiten andererseits;
- Fig. 10: ein Diagramm mit einem Vergleich zwischen einer mittels des erfindungsgemäßen Verfahrens gemessenen Leitfähigkeit einerseits mit kommerziellen Messgeräten gemessenen Leitfähigkeiten andererseits.

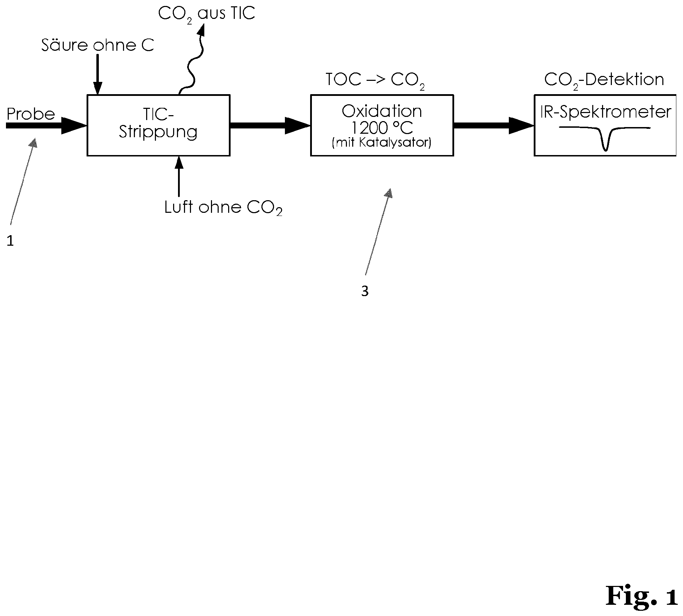

Fig. 1 zeigt ein Ablaufdiagramm betreffend eine Darstellung eines aus dem Stand der Technik bekannten aufwendigen Verfahrens mit einer Kombination aus TIC-Strippung, katalytischer Oxidation und spektroskopischem Nachweis. Die Probe durchläuft bei diesem zunächst Ansäuerungsstufe mit Strippung des dabei ausgasenden CO₂. Es gilt die Anforderung der CO₂-Freiheit an die Strippluft und eine hohe Reinheitsanforderung an die Säure. Anschließend durchläuft die Probe einen auf 1200 °C aufgeheizten Ofen mit Katalysator durchgeführt, schließlich wird der CO₂-Gehalt per IR-Spektrometer ermittelt und daraus der TOC-Gehalt bestimmt.

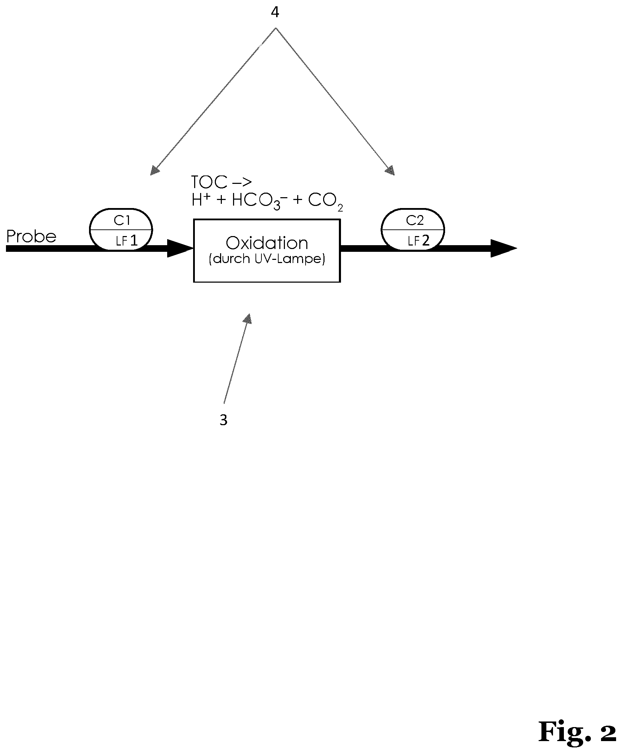

Fig. 2 zeigt ein Ablaufdiagramm betreffend die einfachste und aus dem Stand der Technik bekannte Methode zur Bestimmung des TOC-Gehalts, nämlich mittels Bestimmung der Differenzleitfähigkeit der wässrigen Lösung 1 mit zwischenzeitlicher Oxidation per UV-Lampe. Zunächst wird dabei an einer ersten Messposition C1 einer Messeinrichtung 4 eine erste Leitfähigkeit LF1 der Probe ermittelt. Danach durchläuft die Probe eine Oxidationseinrichtung 3, in welcher die Oxidation von in der wässrigen Lösung 1 enthaltenem organischem Kohlenstoff zu CO₂, H⁺ und HCO₃⁻ mittels UV-Lampe erfolgt. An einer zweiten Messposition C2 der Messeinrichtung 4 wird anschließend eine zweite Leitfähigkeit LF₂ der Probe gemessen. Aus der Differenz der gemessenen Leitfähigkeiten LF₁ und LF₂ wird schließlich der TOC-Gehalt der Probe bestimmt.

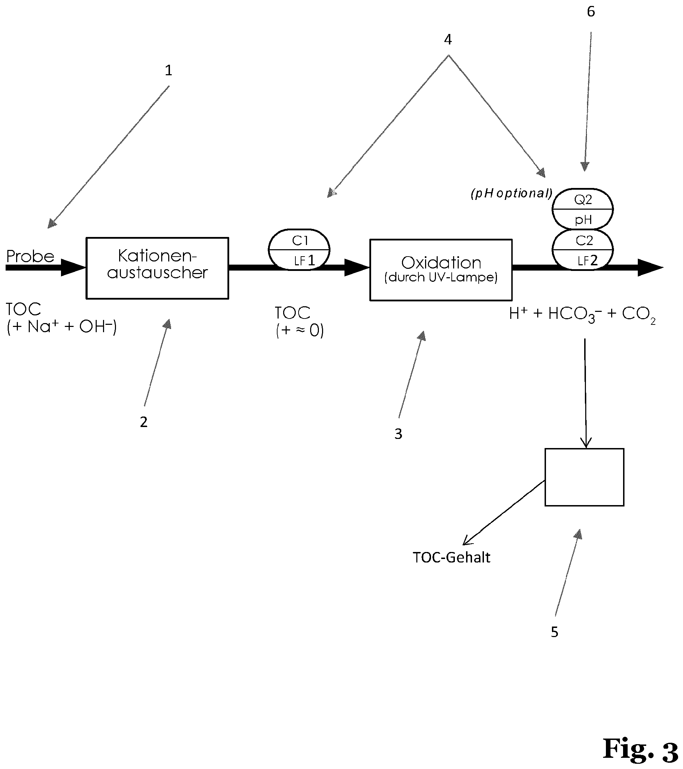

Fig. 3 zeigt ein Ablaufdiagramm einer Ausführungsform des erfindungsgemäßen Verfahrens. Die wässrige Lösung 1, insbesondere ein leicht alkalisches Probenwasser, welches z.B. der Ablauf eines starkbasischen Anionenaustauschers (SBA) einer Ionenaustauscher-Vollentsalzungsstraße sein kann und zu diesem Zeitpunkt noch unerwünschte Anteile von NaOH⁻- und/oder NaHCO₃ aufweist, durchfließt einen Kationenaustauscher 2 in H-Form. Dies kann erfindungsgemäß sowohl ein starksaurer (SAC) aber auch ein schwachsaurer Kationenaustauscher sein (WAC). Durch den Kationenaustauscher 2 werden alle anorganischen Kationen wie z.B. Na⁺, K⁺ usw. gegen H⁺-Ionen ausgetauscht. Diese wiederum neutralisieren die in den leicht alkalischen Abläufen von VE-Straßen enthaltenen OH⁻-Ionen. Die Tatsache, dass die freigesetzten H⁺-Ionen durch die Neutralisation aus dem Ionenaustausch-Gleichgewicht entfernt werden, ermöglicht eine nahezu vollständige Aufnahme der Na⁺-Ionen mit sehr geringen Restwerten ≤ 1 µmol/l. Ebenso ermöglicht diese Neutralisation den Einsatz sowohl von SAC- als auch WAC-Harztypen. Anschließend durchläuft die wässrige Lösung wie in Fig. 2 gezeigt zwei aufeinanderfolgende Leitfähigkeitsmessungen an Messpositionen C1 und C2 einer Messeinrichtung 4 mit dazwischen erfolgender Oxidation mittels UV-Lampe durch eine Oxidationseinrichtung 3. In der gezeigten Ausführungsform wird auf Höhe der zweiten Messposition C2 außerdem an einer dritten Messposition Q2 mittels einer Einrichtung 6 zum Messen des pH-Werts eine pH-Wert-Messung durchgeführt. In einer Auswerteinheit 5 wird wie nachfolgend beschrieben schließlich der TOC-Gehalt aus den gemessenen Leitfähigkeiten LF1 und LF2 sowie dem gemessenen pH-Wert ermittelt. Die typisch gemessene Rest-Leitfähigkeit LF1 an der Messposition C1 beträgt ca. 0,06 bis 0,2 µS/cm, vorrangig 0,07 bis 0,15 µS/cm, was gegenüber gemessenen Werten von 0,5 bis 2 µS/cm bei dem aus dem Stand der Technik bekannten Verfahren der UV-Oxidation ohne vorherigen Kationenaustausch nur noch ein kleiner Störbeitrag ist. Die Testmessungen haben gezeigt, dass die bei der wässrigen Lösung gemessenen pH-Werte nach Durchlaufen der UV-Oxidation mit den aus der Leitfähigkeit theoretisch errechenbaren pH-Werten im Rahmen der erreichbaren pH-Kalibriergenauigkeit von ±0,05 relativ gut übereinstimmen. Diese Korrelation ermöglicht, weitere Korrekturrechnungen durch Mittelwertbildung der beiden Methoden der pH-Wert-Ermittlung durchzuführen, um die Genauigkeit der Berechnung der H⁺-Konzentration zu erhöhen. Die pH-Messung kann somit zur weiteren Verbesserung der Ermittlung der Leitfähigkeitsdifferent mitgenutzt werden, wobei das Ergebnis jedoch auch bereits ohne die zusätzliche pH-Messung sehr genau ist.

Die UV-Lampe der Oxidationseinrichtung 3 ist eine 11 W Niederdrucklampe mit starken Emissionslinien bei 254 und 185 nm. Diese beiden Linien erzeugen insbesondere OH-Radikale im Wasser, welche dann die eigentliche Oxidationsreaktion verursachen.

Die Auswertungsformel kann aufgrund der erfindungsgemäßen Ausführung des Messaufbaus mit einem Kationenaustauscher in guter Näherung dadurch beschrieben werden, dass die wässrige Lösung 1 nach Durchlaufen der UV-Oxidation an den in Fig. 3 gezeigten Messpositionen C2 und Q2 folgende Bestandteile enthält:
- HCO₃⁻ (mit einem spezifischen Leitfähigkeitsbeitrag von ca. 36 (µS/cm)/(mmol/l)
- H⁺ (mit einem spezifischen Leitfähigkeitsbeitrag von 336 (µS/cm)/(mmol/l))
- Na⁺ (Konzentration meist < < 5 µmol/l, spezifische Leitfähigkeit 45 (µS/cm)/(mmol/l))
- OH⁻ (Konzentration meist << 5 µmol/l, spezifische Leitfähigkeit ≈200 (µS/cm)/(mmol/l))
- CO₂ (ohne Leitfähigkeitsbeitrag)

Folgende Rechenschritte können dabei in der Erfindung enthalten sein:
- Zuerst wird die millimolare Konzentration von Na⁺ und OH⁻ vor der UV-Zelle durch Division der Leitfähigkeit C1 durch die Summe der Einzelleitfähigkeiten von Na⁺ und OH⁻ (45+200) µS/cm/(meq/l) ermittelt.
- Dann verbleibt ein Leitfähigkeitsbeitrag für H⁺ + HCO₃⁻ von (336+36) µS/cm/(mmol/l) über den die gemessene und um 0,055 µS/cm verringerte Leitfähigkeit C2 nach der UV-Zelle in die millimolare HCO3⁻ Konzentration linear umgerechnet werden kann. Diese millimolare Konzentration ist die gleiche wie für H⁺, so dass der theoretische pH-Wert daraus einfach zu errechnen ist. Er kann dann mit dem gemessenen pH-Wert verglichen werden. In der Praxis bedeutet das, dass zwei diversitär redundante Messmethoden für die H+-Konzentration genutzt werden können.
- Zuletzt kann über die Henderson-Hasselbalch-Gleichung (HH-Gl.) der Quotient CO₂/HCO₃⁻ aus dem aus der Leitfähigkeit ermittelten oder dem gemessenen oder einem aus beiden gemittelten pH-Wert errechnet werden. Die Gesamt-Kohlensäure ist dann die Summe aus HCO₃⁻ (aus der Leitfähigkeit) und CO₂ (aus HH-Gl.) Die Gesamt-Kohlensäure in mmol/l wird dann durch Multiplikation der Molmasse von Kohlenstoff (12000 µg/mmol) in den TOC-Wert in µg/l = ppb umgerechnet.

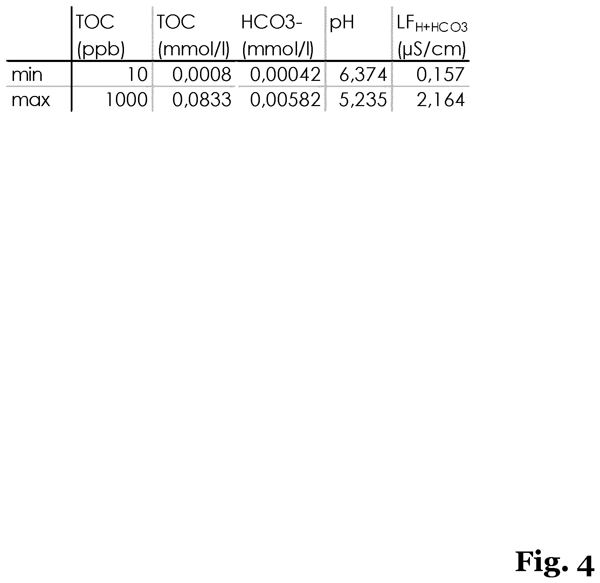

Diese Rechenmethode entspricht den in Fig. 4 gezeigten Werten in den Spalten von rechts nach links aus betrachtet. Die Erwartungswerte für die durch Oxidation des TOC zusätzlich entstandene Kohlensäure und die messbaren Werte für Leitfähigkeit und pH nach Durchlaufen der UV-Zelle sind dabei für die angestrebten Messbereichsenden 10 und 1000 ppb dargestellt. Die in dieser Tabelle rechts aufgeführten kleinsten und größten Messwerte für LF_{H+HCO3} (welche der Leitfähigkeit LF2 an der Messposition C2 nach UV-Oxidation entspricht) sind tatsächlich in einer Größenordnung, welche den Messwerten im Ablauf einer VE-Straße direkt entspricht. Dies zeigt das Grundproblem der Leitfähigkeits-basierten TOC-Messtechnik, welche aber durch das erfindungsgemäße Vorgehen wirksam behoben werden kann. Die Gültigkeit der Berechnung des nicht messbaren CO₂-Anteils über den pH-Wert aus dem messbaren HCO₃⁻-Anteil kann über die Übereinstimmung von theoretischem pH-Wert (aus der Leitfähigkeit errechnet) mit dem gemessenen pH-Wert überprüft werden (was in der Fig. 3 als optionale aber erfindungsgemäß nicht notwendige Ergänzung gezeigt ist). Diese Fehlerbetrachtung wird im folgenden Abschnitt als Nachweis der Wirksamkeit der Erfindung beschrieben.

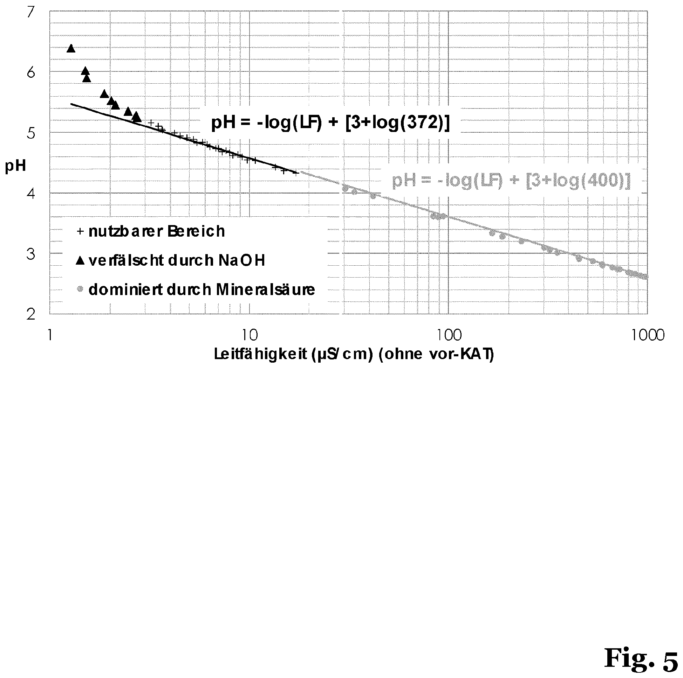

Der Zusammenhang zwischen Leitfähigkeit und pH-Wert gehorcht für Säuren einem einfachen logarithmischen Gesetz, welches in Fig. 5 dargestellt ist. Diese weist diverse Messpunkte von in einem Ablauf eines Anionenaustauschers gemessenen pH-Werten auf. Das Diagramm überdeckt einen großen Bereich von durch eine VE-Straße schlupfenden NaOH und Säuren. Im Diagramm der Fig. 5 sind drei Bereiche erkennbar: Links in Form von Dreiecken dargestellt ist der durch NaOH deutlich verfälschte Gültigkeitsbereich der TOC-Berechnung ohne den erfindungsgemäßen Kationenaustauscher. Wie zu erkennen ist, weichen die Dreiecke deutlich von der Ausgleichsgeraden nach oben ab und stören die Berechnungsmethode. Im mittleren Bereich, welcher in Form von schwarzen "+"-Zeichen verdeutlicht ist, liegt die kleinste noch regelgerecht umrechenbare Leitfähigkeit bei ca. 3 µS/cm, also im Bereich der schwarzen "+" Zeichen, welche noch nicht nach oben von der Geraden abwandern. Dieser Wert entspräche ca. 2000 ppb TOC, was für die erfindungsgemäße Anwendung demnach ein vollkommen unbrauchbarer Bereich ist. Leicht ist jedoch erkennbar, dass die erfindungsgemäße und erreichte Absenkung der Leitfähigkeits-Störanteile auf ≈ 0,1 µS/cm den im Sinne der beabsichtigten Messmethode linear auswertbaren Bereich der schwarzen "+"-Zeichen, auf bis deutlich unter 1 µS/cm erweitern würde, wie durch die oben erwähnte nach links weitergeführte Ausgleichsgerade dargestellt ist. Der rechte graue Anteil ist für die vorliegende Aufgabe nicht mehr interessant, da er nur den durch Mineralsäuren dominierten Bereich anzeigt, in dem Kohlensäure demnach ebenfalls nicht mehr gemessen werden kann. Es ist erkennbar, dass eine Unterscheidung zwischen Kohlensäure und Mineralsäure, welche nur über den kleinen Unterschied im additiven Anteil möglich wäre, der durch den kleinen Versatz der beiden Ausgleichsgeraden im Bereich von ca. 20 µS/cm leicht erkennbar ist, bei den vorliegenden Messungenauigkeiten nicht funktioniert. Es muss also vorausgesetzt werden, dass die Probenlösung keine Mineralsäuren enthält, was aber für die Abläufe von VE-Straßen in der Regel zutrifft. Im Diagramm der Fig. 5 wurde zur Ermittlung der Datenpunkte der Anionenaustauscher deutlich über die Kapazitätsgrenze hinaus überfahren und produzierte dabei nicht bestimmungsgemäß diese mineralsauren Abläufe.

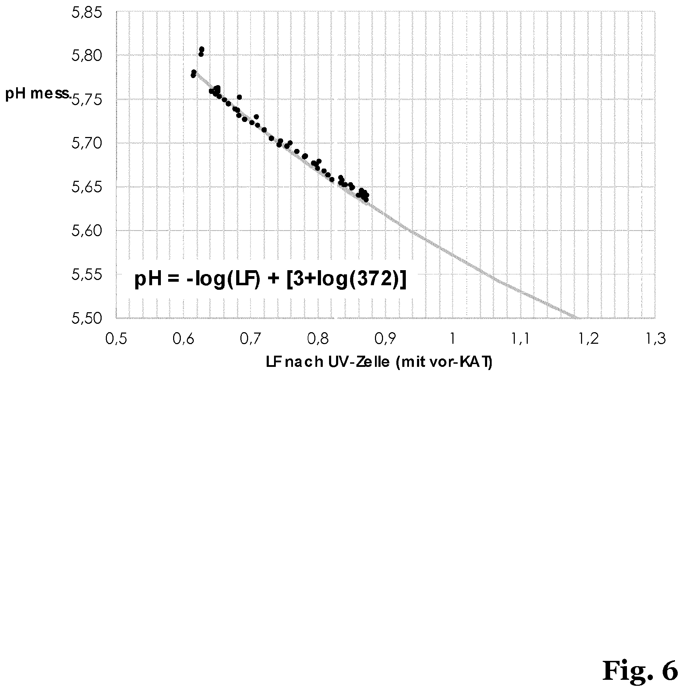

Der Erfolg des vorgeschalteten Kationenaustauschers (vor-KAT) 2 ist im Diagramm in Fig. 6 gezeigt, welche verschiedene Messpunkte durch variierende TOC-Anteile gemessen hinter der UV-Zelle bzw. Oxidationseinrichtung 3 an den Messpositionen Q2 und C2 zeigt. Insbesondere ist hierbei der sehr niedrige Leitfähigkeits-Messbereich auf der X-Achse des Diagramms zu beachten.

Sehr deutlich ist zu erkennen, wie gut die theoretische Beziehung zwischen Leitfähigkeit und pH-Wert selbst bei < 1 µS/cm noch funktioniert, und wie gut die Unterdrückung von Einflüssen der NaOH in der Originalprobe durch den erfindungsgemäßen vor-KAT 2 ist. Es ist zu beachten, dass hier sogar eine lineare X-Achse gewählt wurde, welche Abweichungen noch deutlicher zeigt, aber im Gegensatz zu Fig. 5 eine gekrümmte logarithmische Anpassungskurve bewirkt. Diese Grafik zeigt also sehr gut, dass der erfindungsgemäße vor-KAT 2 das den Stand der Technik abbildende Diagramm in Fig. 5 tatsächlich wie erwartet sehr weit zu kleineren Leitfähigkeiten erweitert und dadurch die gewünschten Nachweisgrenzen für den TOC erreichen kann.

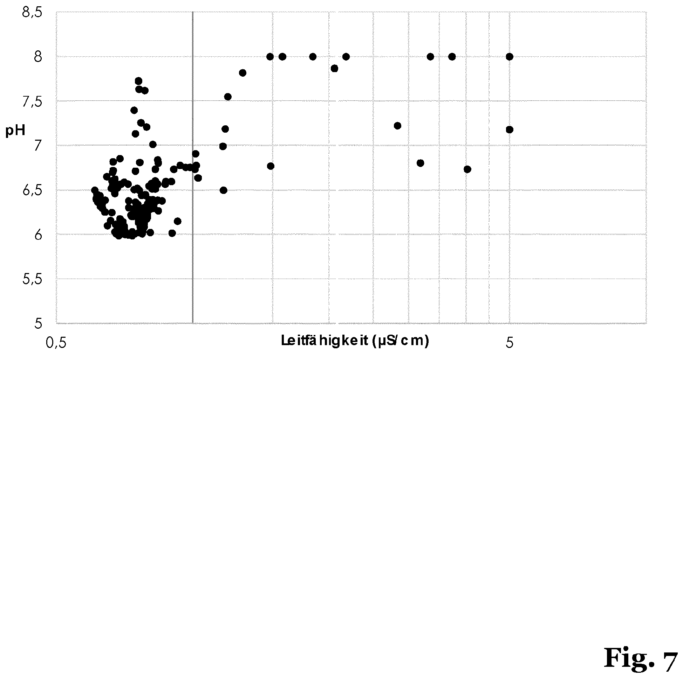

In praktischen Messungen zeigen sich noch weitere deutliche Verbesserungen der Auswertbarkeit der Leitfähigkeitsmessungen nach der UV-Zelle bzw. der Oxidationseinrichtung 3 durch den erfindungsgemäß vor der UV-Zelle 3 angeordneten Kationenaustauscher 2. Fig. 7 zeigt Messpunkte in einem Diagramm in der gleichen Darstellungsweise wie in Fig. 5 oder Fig. 6. ohne die Verwendung des vorgeschalteten Kationenaustauschers 2. Die Messungen sind alle aus dem leicht über Na schlupfenden also leicht alkalischen Ablauf einer VE-Straße und nach der UV-Zelle an der zweiten Messposition C2 aufgenommen. Hierbei wurde der Anionenaustauscher der VE-Straße aber nicht - wie in den Fig. 5 und Fig. 6 - über den Säuredurchbruch hinaus überfahren, sondern befand sich noch im optimalen Arbeitsbereich vor dem Beladeende der VE-Straße. Sehr deutlich ist dabei zu erkennen, dass die nach der Korrelation aus den Fig. 5 und 6 zu erwartende Linie der Punkte überhaupt nicht zu erkennen ist. Es ist sogar eher ein Trend mit positiver Steigung zu erahnen, der aufzeigt, dass noch NaOH-Reste vorhanden sind. Immerhin sind die pH-Werte dort auch dementsprechend hoch. Jedoch zeigt sich, dass das Diagramm einen Satz von Messpunkten zeigt, welcher so nicht auswertbar ist.

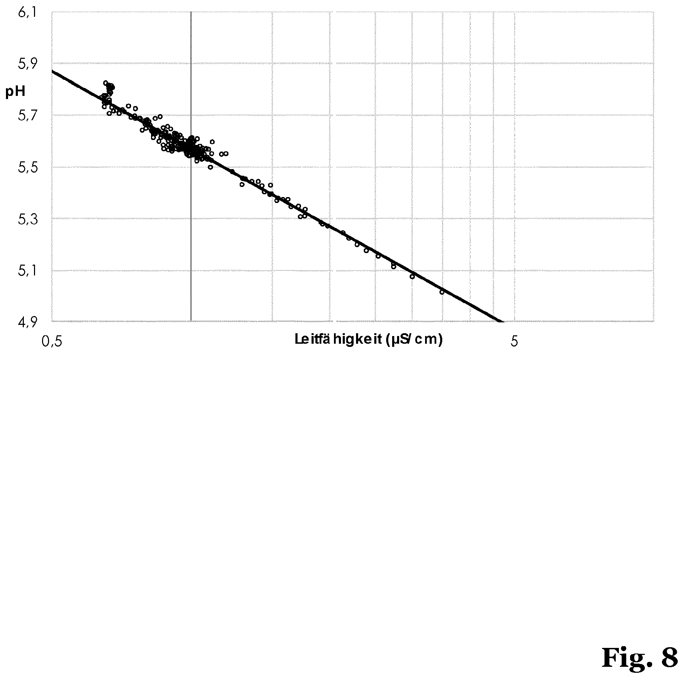

In einem weiteren Versuchslauf wurde anschließend der erfindungsgemäße Kationenaustauscher verwendet und die gleiche Auftragung von pH-Wert gegenüber der Leitfähigkeit an den Messpositionen C2 bzw. Q2 dargestellt, wie Fig. 8 zeigt. Auf den ersten Blick ist dabei erkennbar, wie nun die Punkte exakt der theoretischen Geraden für Kohlensäure entsprechen, welche durch die schwarze Linie mit den theoretischen Leitfähigkeitsbeiträgen für H⁺ und HCO₃⁻ verdeutlicht ist.

Der Vergleich zwischen den Diagrammen der Fig. 7 und der Fig. 8 verdeutlichen noch einmal sehr eindeutig, wie der erfindungsgemäße Kationenaustauscher für die Auswertbarkeit von Leitfähigkeitsmessungen die bekannten theoretischen Formeln ohne empirische Korrekturen nutzbar macht. Eine Schlussfolgerung daraus ist der nicht zu unterschätzende Vorteil der Erfindung, dass diese TOC-Messung überhaupt keine Kalibration mit TOC-Maßlösungen benötigt. Die Leitfähigkeitsmessungen können mit einfachen Mitteln justiert werden. Sonst ist keine Kalibration oder Justierung mehr nötig, da wie oben erläutert, alles über bekannte theoretische Zusammenhänge und Formeln errechenbar ist. Im Gegensatz dazu werden bei den zum Vergleich verwendeten Analysengeräten immer teure Kalibrationslösungen mit definierten TOC-Gehalten verwendet, um die Geräte regelmäßig zu justieren.

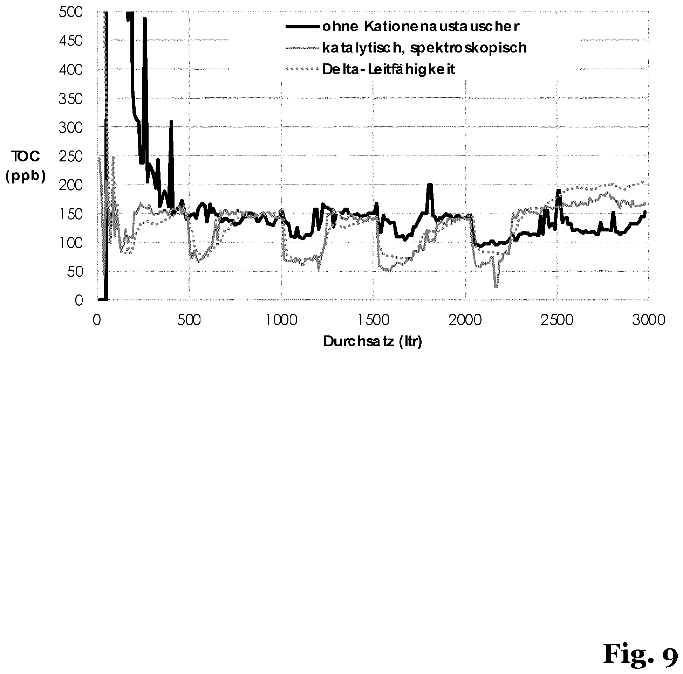

Ein Diagramm mit TOC-Kurven ohne Einsatz des erfindungsgemäßen Kationenaustauschers ist in Fig. 9 gezeigt. Hier wurden neben der hier beschriebenen UV-Oxidationszelle 3 mit Leitfähigkeitsmessung an der Messposition C2 ohne vorgeschaltetem Kationenaustauscher auch noch zwei kommerzielle Analysengeräte verwendet, welche nach ihrem Messprinzip in Fig. 9 benannt sind. Beide kommerziellen Analysengeräte mussten dabei allerdings aufwendig mit Kalibrierlösungen justiert werden. Sehr deutlich ist zu erkennen, dass zwar eine großräumige Entsprechung der schwarzen Kurve (ohne erfindungsgemäßem Kationenaustauscher) mit den beiden grauen Referenzkurven vorliegt, eine Entsprechung zu den feineren Variationen der Referenzkurven ist hingegen nicht vorhanden.

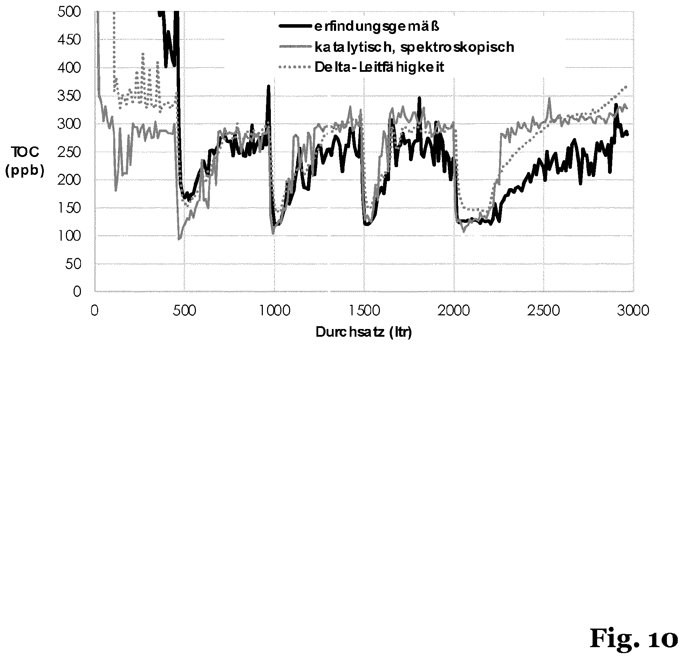

Der in Fig. 10 dargestellte Kurvensatz zeigt als schwarze Kurve das Messergebnis beim erfindungsgemäßen Aufbau mit Kationenaustauscher 2 und nachgeordneter UV-Zelle 3 mit folgender Leitfähigkeitsmessung an der Messposition C2. Die beiden grauen Kurven zeigen wieder die Messdaten der kommerziellen Referenzgeräte. In Fig. 10 ist gegenüber Fig. 9 auf den ersten Blick die sehr deutliche Verbesserung der Entsprechung der Kurven erkennbar. Es ist sogar ablesbar, dass die Abweichung der schwarzen Kurve relativ zu den grauen Kurven in einer ähnlichen Größenordnung liegt, wie die Abweichungen der beiden grauen Referenzkurven untereinander. Dies bedeutet, dass die erfindungsgemäße Messanordnung durch die hier beschriebene Verbesserung qualitativ mit vielfach teureren kommerziellen Messgeräten in Konkurrenz treten kann.

### Bezugszeichenliste

- 1: wässrige Lösung
- 2: Austauscheinrichtung
- 3: Oxidationseinrichtung
- 4: Messeinrichtung
- 5: Auswerteinheit
- 6: Einrichtung zum Messen des pH-Werts
- C1: erste Messposition
- C2: zweite Messposition
- LF1: erste Leitfähigkeit
- LF2: zweite Leitfähigkeit
- Q2: dritte Messposition

## Patentansprüche

1. Verfahren zum Bestimmen des TOC-Gehaltes einer wässrigen Lösung (1) nach Durchlaufen einer Ionenaustauscher-Vollentsalzungsstraße, aufweisend die Schritte:
a) Messen einer ersten Leitfähigkeit (LF1) der wässrigen Lösung (1);
b) Oxidieren von in der wässrigen Lösung (1) enthaltenem organischen Kohlenstoff zu CO₂ und/oder H⁺ und/oder HCO₃⁻ und/oder Na⁺ + HCO₃⁻;
c) Messen einer zweiten Leitfähigkeit (LF2) der wässrigen Lösung (1);
d) Bestimmen des TOC-Gehalts der wässrigen Lösung aus den beiden gemessenen Leitfähigkeiten (LF1, LF2),
**dadurch gekennzeichnet, dass** vor dem Messen der ersten Leitfähigkeit (LF1) ein Austauschen von in der wässrigen Lösung enthaltenen anorganischen Kationen, wie beispielsweise Na⁺, K⁺ usw., gegen H⁺-Ionen erfolgt, wobei das Austauschen ein Umwandeln des NaOH⁻- und/oder des NaHCO₃-Anteils der wässrigen Lösung (1) in H₂O und CO₂ mittels eines Kationenaustauschers (2) in H⁺-Form umfasst.

2. Verfahren nach Anspruch 1, wobei mittels des Kationenaustauschers (2) das Na⁺ in der wässrigen Lösung (1) gegen H⁺ ausgetauscht wird, welches überschüssige OH⁻ -Ionen zu Wasser neutralisiert.

3. Verfahren nach einem der vorangehenden Ansprüche, wobei die wässrige Lösung (1) nach dem Oxidieren Konzentrationen von Na⁺ und/oder OH⁻ von jeweils geringer als 5 µmol/l aufweist.

4. Verfahren nach einem der Ansprüche 1 bis 3, wobei der Kationenaustauscher (2) ein starksaurer oder ein schwachsaurer Kationenaustauscher ist.

5. Verfahren nach einem der vorangehenden Ansprüche, wobei das Oxidieren des in der wässrigen Lösung (1) enthaltenen organischen Kohlenstoffs mittels UV-Bestrahlung erfolgt.

6. Verfahren nach Anspruch 5, wobei die UV-Bestrahlung mittels zumindest einer Niederdruck-UV-Lampe erfolgt.

7. Verfahren nach einem der vorangehenden Ansprüche, wobei beim Messen der ersten und der zweiten Leitfähigkeit (LF1, LF2) der wässrigen Lösung (1) jeweils die Eigenleitfähigkeit von H₂O herausgerechnet wird.

8. Verfahren nach einem der vorangehenden Ansprüche, ferner umfassend das Messen des pH-Werts der wässrigen Lösung (1) nach dem Oxidieren des in der wässrigen Lösung (1) enthaltenen organischen Kohlenstoffs;
Berechnen eines theoretischen pH-Werts aus der gemessenen zweiten Leitfähigkeit (LF₂);
Bilden eines Mittelwerts aus dem gemessenen pH-Wert und dem berechneten theoretischen pH-Wert;
Verwenden des gemittelten pH-Werts für die Berechnung der Leifähigkeitsdifferenz.

9. Vorrichtung zum Bestimmen des TOC-Gehaltes einer wässrigen Lösung nach Durchlaufen einer Ionenaustauscher-Vollentsalzungsstraße,
mit zumindest einer Messeinrichtung (4) zum Messen der Leitfähigkeit der wässrigen Lösung (1), welche eine erste Messposition (C1) zum Messen einer ersten Leitfähigkeit (LF1) und eine zweite Messposition (C2) zum Messen einer zweiten Leitfähigkeit (LF2) der wässrigen Lösung (1) aufweist, wobei die erste Messposition (C1) stromaufwärts der zweiten Messposition (C2) angeordnet ist, und mit einer zwischen den Messpositionen (C1, C2) angeordneten Oxidationseinrichtung (3), die vorzugsweise zumindest eine Niederdruck-UV-Lampe aufweist, zum Oxidieren von in der wässrigen Lösung (1) enthaltenem organischen Kohlenstoff zu CO₂ und/oder H⁺ und/oder HCO₃⁻ und/oder Na⁺ + HCO₃⁻, sowie mit einer Auswerteinheit (5) zum Berechnen der Differenz zwischen der ersten und der zweiten gemessenen Leitfähigkeit (C1, C2) und zum Bestimmen des TOC-Gehalts der wässrigen Lösung (1) aus der berechneten Leitfähigkeitsdifferenz,
**dadurch gekennzeichnet, dass** die Vorrichtung ferner einen stromaufwärts der ersten Messposition (C1) angeordneten Kationenaustauscher in H⁺-Form (2) zum Austauschen von in der wässrigen Lösung (1) enthaltenen anorganischen Kationen, wie beispielsweise Na+, K+ usw., gegen H+-Ionen aufweist, bevorzugt der Kationenaustauscher ein starksaurer oder ein schwachsaurer Kationenaustauscher ist.

10. Vorrichtung nach Anspruch 9, ferner umfassend eine stromabwärts der Oxidationseinrichtung (3) angeordnete Einrichtung (6) zum Messen des pH-Werts der wässrigen Lösung (1) aufweist, wobei die Auswerteinheit (5) ferner zum Berechnen eines theoretischen pH-Werts aus der gemessenen zweiten Leitfähigkeit (C2), zum Bilden eines Mittelwerts aus dem gemessenen pH-Wert und dem berechneten theoretischen pH-Wert sowie zum Verwenden des gemittelten pH-Werts für die Berechnung der Leifähigkeitsdifferenz eingerichtet ist.

11. Vorrichtung nach einem der Ansprüche 9 oder 10, ferner aufweisend eine stromaufwärts der Austauscheinrichtung (2) angeordnete Ionenaustauscher-Vollentsalzungsstraße.

## Claims

1. Method for determining the TOC content of an aqueous solution (1) after passing through an ion exchanger complete desalination line, comprising the steps:
a) Measuring a first conductivity (LF1) of the aqueous solution (1);
b) Oxidizing organic carbon contained in the aqueous solution (1) to CO₂ and/or H⁺ and/or HCO₃⁻ and/or Na⁺ + HCO₃⁻;
c) Measuring a second conductivity (LF2) of the aqueous solution (1);
d) Determining the TOC content of the aqueous solution from the two measured conductivities (LF1, LF2),
**characterized in that** prior to measuring the first conductivity (LF1), exchanging inorganic cations contained in the aqueous solution, such as Na⁺, K⁺, etc., for H⁺ ions takes place, wherein the exchange includes conversion of the NaOH⁻ and/or NaHCO₃ portion of the aqueous solution (1) into H₂O and CO₂ by means of a cation exchanger (2) in H⁺ form.

2. Method according to claim 1, wherein the cation exchanger (2) exchanges Na⁺ in the aqueous solution (1) for H⁺, which neutralizes excess OH⁻ ions to water.

3. Method according to any one of the preceding claims, wherein the aqueous solution (1) after oxidation has concentrations of Na⁺ and/or OH⁻ each lower than 5 µmol/l.

4. Method according to any one of claims 1 to 3, wherein the cation exchanger (2) is a strongly acidic or a weakly acidic cation exchanger.

5. Method according to any one of the preceding claims, wherein the oxidation of the organic carbon contained in the aqueous solution (1) is performed by means of UV irradiation.

6. Method according to claim 5, wherein the UV irradiation is performed by at least one low-pressure UV lamp.

7. Method according to any one of the preceding claims, wherein during the measurement of the first and second conductivity (LF1, LF2) of the aqueous solution (1), the intrinsic conductivity of H₂O is subtracted.

8. Method according to any one of the preceding claims, additionally comprising measuring the pH value of the aqueous solution (1) after oxidizing the organic carbon contained in the aqueous solution (1); calculating a theoretical pH value from the measured second conductivity (LF2); forming an average from the measured pH value and the calculated theoretical pH value; using the averaged pH value to calculate the conductivity difference.

9. Apparatus for determining the TOC content of an aqueous solution after passing through an ion exchanger complete desalination line, comprising at least one measuring device (4) for measuring the conductivity of the aqueous solution (1), which has a first measuring position (C1) for measuring a first conductivity (LF1) and a second measuring position (C2) for measuring a second conductivity (LF2) of the aqueous solution (1), wherein the first measuring position (C1) is arranged upstream of the second measuring position (C2), and an oxidation device (3) arranged between the measuring positions (C1, C2), which preferably comprises at least one low-pressure UV lamp, for oxidizing organic carbon contained in the aqueous solution (1) to CO₂ and/or H⁺ and/or HCO₃^{~} and/or Na⁺ + HCO₃¯, and an evaluation unit (5) for calculating the difference between the first and second measured conductivities (C1, C2) and for determining the TOC content of the aqueous solution (1) from the calculated conductivity difference,
**characterized in that** the apparatus further comprises a cation exchanger in H⁺ form (2) arranged upstream of the first measuring position (C1) for exchanging inorganic cations contained in the aqueous solution (1), such as Na⁺, K⁺, etc., for H⁺ ions, preferably the cation exchanger is a strongly acidic or a weakly acidic cation exchanger.

10. Apparatus according to claim 9, further comprising a device (6) arranged downstream of the oxidation device (3) for measuring the pH value of the aqueous solution (1), wherein the evaluation unit (5) is further configured to calculate a theoretical pH value from the measured second conductivity (C2), to form an average from the measured pH value and the calculated theoretical pH value, and to use the averaged pH value for calculating the conductivity difference.

11. Apparatus according to any of claims 9 or 10, further comprising an ion exchange complete desalination stage arranged upstream of the exchange device (2)

## Revendications

1. Procédé de détermination de la teneur en COT d'une solution aqueuse (1) après passage dans une chaîne de déminéralisation complète par échange d'ions, comprenant les étapes suivantes :
a) Mesure d'une première conductivité (LF1) de la solution aqueuse (1) ;
b) Oxydation du carbone organique contenu dans la solution aqueuse (1) en CO₂ et/ou H⁺ et/ou HCO₃⁻ et/ou Na⁺ + HCO₃⁻ ;
c) Mesure d'une seconde conductivité (LF2) de la solution aqueuse (1) ;
d) Détermination de la teneur en COT de la solution aqueuse à partir des deux conductivités mesurées (LF1, LF2),
**caractérisé en ce que**, avant la mesure de la première conductivité (LF1), les cations minéraux contenus dans la solution aqueuse, tels que Na⁺, K⁺, etc., sont échangés contre des ions H⁺, l'échange comprenant la conversion des fractions NaOH et/ou NaHCO₃ de la solution aqueuse (1) en H₂O et CO₂ au moyen d'un échangeur de cations (2) sous forme H⁺.

2. Procédé selon la revendication 1, l'échangeur de cations (2) permettant d'échanger le Na⁺ de la solution aqueuse (1) contre des H⁺, lesquels neutralisent les ions OH⁻ excédentaires pour former de l'eau.

3. Procédé selon l'une des revendications précédentes, la solution aqueuse (1) présentant après oxydation des concentrations de Na⁺ et/ou OH⁻ chacune inférieure à 5 µmol/l.

4. Procédé selon l'une des revendications 1 à 3, l'échangeur de cations (2) étant un échangeur de cations fortement acide ou faiblement acide.

5. Procédé selon l'une des revendications précédentes, l'oxydation du carbone organique contenu dans la solution aqueuse (1) étant réalisée par irradiation UV.

6. Procédé selon la revendication 5, l'irradiation UV étant réalisée au moyen d'au moins une lampe UV basse pression.

7. Procédé selon l'une des revendications précédentes, la mesure des première et seconde conductivités (LF1, LF2) de la solution aqueuse (1) incluant la soustraction de la conductivité propre de l'eau (H₂O).

8. Procédé selon l'une des revendications précédentes, comprenant en outre la mesure du pH de la solution aqueuse (1) après oxydation du carbone organique ; calcul d'un pH théorique à partir de la seconde conductivité mesurée (LF2) ; détermination d'une valeur moyenne entre le pH mesuré et le pH théorique calculé ; utilisation de la valeur moyenne du pH pour le calcul de la différence de conductivité.

9. Dispositif pour déterminer la teneur en COT d'une solution aqueuse après passage dans une chaîne de déminéralisation complète par échange d'ions,
comprenant au moins un dispositif de mesure (4) pour mesurer la conductivité de la solution aqueuse (1), comportant une première position de mesure (C1) pour la mesure de la première conductivité (LF1) et une seconde position de mesure (C2) pour la mesure de la seconde conductivité (LF2), la première position de mesure (C1) étant disposée en amont de la seconde position de mesure (C2),
et comprenant un dispositif d'oxydation (3), positionné entre les positions de mesure (C1, C2) et comprenant de préférence au moins une lampe UV basse pression, pour l'oxydation du carbone organique contenu dans la solution aqueuse (1) en CO₂ et/ou H⁺ et/ou HCO₃⁻et/ou Na⁺ + HCO₃⁻, ainsi qu'une unité d'évaluation (5) pour calculer la différence entre les première et seconde conductivités mesurées (C1, C2) et déterminer la teneur en COT à partir de cette différence,
**caractérisé en ce que** le dispositif comprend en outre un échangeur de cations sous forme H⁺ (2), disposé en amont de la première position de mesure (C1) pour échanger les cations minéraux contenus dans la solution aqueuse (1), tels que Na⁺, K⁺, etc., contre des ions H⁺, l'échangeur de cations étant de préférence un échangeur fortement acide ou faiblement acide.

10. Dispositif selon la revendication 9, comprenant en outre un dispositif (6) disposé en aval du dispositif d'oxydation (3) pour mesurer le pH de la solution aqueuse (1), l'unité d'évaluation (5) étant en outre configurée pour calculer un pH théorique à partir de la seconde conductivité mesurée (C2), déterminer une moyenne entre le pH mesuré et le pH théorique calculé, et utiliser la valeur moyenne du pH pour calculer la différence de conductivité.

11. Dispositif selon l'une des revendications 9 ou 10, comprenant en outre une chaîne de déminéralisation complète par échange d'ions disposée en amont de l'échangeur (2).
